# EUROPEAN PATENT APPLICATION

(11) **EP 4 523 677 A1**
(43) Date of publication of application: **19.03.2025**
(21) Application number: 23197079.9
(22) Date of filing: 13.09.2023
(51) Int. Cl.: A61K 9/00, A61K 9/06, A61K 47/36

(54) **TOPICAL COMPOSITION OF NADIFLOXACIN AND BENZOYL PEROXIDE**

(71) Applicant: Dr. Pfleger Arzneimittel GmbH, 96052 Bamberg (DE)
(72) Inventor: KAPOTE, Dnyaneshwar, 96047 Bamberg (DE); DANIELS, Rolf, 72108 Rottenburg (DE); BABIC, Alija, 72070 Tübingen (DE)
(74) Representative: Eisenführ Speiser

(57) **Abstract**

The present invention is in the field of topical pharmaceutical formulations and provides compositions with nadifloxacin and benzoyl peroxide for the treatment of inflammatory skin conditions, in particular acne. Furthermore, the invention provides a method for manufacturing such a topical composition.

## Description

The present invention is in the field of topical pharmaceutical formulations and provides compositions with nadifloxacin and benzoyl peroxide for the treatment of inflammatory skin conditions, in particular acne. Furthermore, the invention provides a method for manufacturing such a topical composition.

The treatment scheme of acne using either the antibiotic nadifloxacin or benzoyl peroxide is known in the literature and is a standard procedure. Several combination therapies of these two active pharmaceutical ingredients (API) are described in literature. For example, firstly, a topical composition of nadifloxacin, mostly in the form of a cream, is used with a typical dosage of 1 wt.-%. In addition, as an add-on therapy, benzoyl peroxide is used (Choudhury S, Chatterjee S, Sarkar DK, Dutta RN. Efficacy and safety of topical nadifloxacin and benzoyl peroxide versus clindamycin and benzoyl peroxide in acne vulgaris: A randomized controlled trial. Indian J Pharmacol. 2011 Nov;43(6):628-31. doi: 10.4103/0253-7613.89815. PMID: 22144763; PMCID: PMC3229774*).*

The mechanism underlying this treatment includes an antibacterial effect of the antibiotic nadifloxacin, which inhibits the bacterial growth by inhibiting the enzyme gyrase. The additional therapy with benzoyl peroxide increases this effect as benzoyl peroxide is a keratolytic medicine, which penetrates the skin and inhibits microbial growth of bacteria associated with acne. Another effect of the combination therapy is that potential resistances to the antibiotic nadifloxacin can be diminished by sequentially administering benzoyl peroxide.

Clindamycin is another antibiotic and belongs to the lincomycin family. It is used in the treatment of acne. Several topical compositions exist, which combine clindamycin and benzoyl peroxide as a combination preparation. Such a composition is e.g. described in the European patent EP 2299810 B1.

A combination of nadifloxacin and benzoyl peroxide as a combination preparation is rather uncommon and has not been present in the EMEA region, as the antibiotic nadifloxacin is highly susceptible to degradation by benzoyl peroxide. Benzoyl peroxide is an instable substance and emits free radicals during its degradation, which either degrade keratin and unblocking the drainage of sebum, but also degrade nadifloxacin.

Thus, special solutions are needed to combine these two APIs. Only one consumer product exists, which is Zinderm BN Gel by Wockhardt Ltd. The degradation issue was solved by incorporating benzoyl peroxide in microspheres, such that it does not come into contact with nadifloxacin and limiting the amount of benzoyl peroxide in the formulation. The incorporation into microspheres is costly and it is not guaranteed that benzoyl peroxide is released from the microspheres when applying the gel to the dermis.

It was thus a primary objective of the present invention to provide a topical composition of nadifloxacin and benzoyl peroxide with a high stability and efficient formulation. Furthermore, a method for manufacturing such a topical composition should be provided.

This primary object was solved by providing a topical composition comprising:
i) nadifloxacin or a pharmaceutically acceptable salt thereof,
ii) benzoyl peroxide,
iii) at least one gel forming agent, preferably selected from the group consisting of xanthan gum, co-polymers of taurate and acrylate, carbomer, modified maize starch, pregelatinised starch, microcrystalline cellulose, guar gum, acrylamido-2-methylpropanesulfonic acid, hydroxyethyl acrylate / sodium acryloyl dimethyl taurate copolymer, polyacrylate crosspolymer-6, crosslinked homopolymer based on 2-acrylamido-2-methylpropane sulfonic acid, copolymer based on 2-acrylamido- 2-methylpropane sulfonic acid and ethoxylated behenyl alcohol methacrylate, crosslinked copolymer based on 2-acrylamido-2-methylpropane sulfonic acid and N-vinylpyrrolidone and copolymer based on acrylamide/sodium acryloyldimethyl taurate /isohexadecane/ polysorbate 80, preferably in an amount of from 0.01 to 10 wt.-%, preferably of from 0.1 to 5 wt.-% and especially preferably in an amount of from 1 to 4 wt.-%, based on the total weight of the topical composition,
iv) at least one buffering agent,
v) at least one moisturizing agent.

Nadifloxacin (CAS-No.: 124858-35-1) is an antibiotic from the fluorquinolone class and inhibits the DNA gyrase. It is a racemic mixture of (R)- and (S)- 9-Fluor-8-(4-hydroxypiperidino)-5-methyl-1-oxo-6,7-dihydro-1*H*,5*H*-benzo[*ij*]chinotizin-2-carbonsäure. It is used exclusively topical in the treatment of skin diseases, especially in the treatment of acne vulgaris.

Preferably, the term "pharmaceutically acceptable salt", as used herein, refers to non-toxic salts. Preferably, the salts are derived from acids such as acetic acid, aconitic acid, citric acid, embolic acid, enanthic acid, hydrobromic acid, hydrochloric acid, lactic acid, maleic acid, malic acid, methanesulphonic acid, phosphoric acid, phthalic acid, salicylic acid, sorbic acid, succinic acid, sulfuric acid, or tartaric acid.

Benzoyl peroxide (or di-benzoylperoxide, CAS-No.: 94-36-0) is an organic peroxide and is used in organic chemistry as radical starter for e.g., radical polymerisation. In dermatology, it is used as an acne therapeutic agent against acne as it is antibacterial, not komedogen, inhibits the proliferation of the sebaceous glands and is assigned to shrink the horn cells of the epidermis.

A "gel forming agent" in terms of the present invention is to be understood as a preferably polymeric substance, which is able to increase the viscosity of a formulation to a thick gel-like texture. When dissolved in water, gel forming agents form a colloidal mixture with a weakly cohesive internal structure. It was surprisingly discovered in terms of the present invention that it is sufficient to include a gel forming agent to decrease the degradation of nadifloxacin by benzoyl peroxide.

A "buffering agent" in terms of the present invention is to be understood as a substance, which is able to maintain a solution in a specific pH range or at a specific pH. A solution containing a buffering agent can, up to a point, absorb the additional hydronium or hydroxide ions that are introduced when an acid or base is added or formed without any alteration to the H₃O⁺/OH⁻ ratio, and thus no change in pH.

A "moisturizing agent" or "moisturizer" in terms of the present invention is to be understood as a substance, which is able to provide or retain water to or in the skin and thus hydrates the skin. In terms of the present invention, preferably the moisturizing agent is a humectant, which belong to the group of hygroscopic substances and attract and retain moisture in the skin.

The gel forming agents according to the invention preferably have a broad pH range, in which they are stable, such that a high pH-dependent decrease in viscosity is avoided, which would lead to an instability of the topical formulation.

It was surprisingly found in context of the present invention that the use of antioxidants does not have an influence on the stability of benzoyl peroxide, which would have been assumed as antioxidants are able to bind free radicals.

Preferably, if a topical composition according to the invention further comprises a lipid phase, preferably, wherein the lipid phase comprises squalane or paraffin.

Furthermore preferably, the topical composition according to the invention further comprises at least one emulsifier selected from the group consisting of glyceryl monostearate, PEG-75 stearate, PEG-6 stearate, ethylene glycol stearate, PEG-32 stearate, cetyl alcohol and ethoxylated fatty alcohols, diisostearate, macrogol 30 dipolyhydroxystearate, triglycerol diisostearate.

Also preferably, the topical composition according to the invention further comprises at least one surfactant selected from the group consisting of caprylocaproyl polyoxyl-8 glycerides, polysorbate 20, polysorbate 60, polysorbate 80, propylene glycol isostearate solubiliser: diethylene glycol monoethyl ether, poloxamer or propylene glycol monolaurate.

Moreover preferably, the topical composition according to the invention further comprises at least one excipient selected from the group consisting of propylene glycol monolaurate, ethylcellulose and propylene glycol isostearate.

Preferably, the lipid phase of the topical composition comprises or consists of 0.01 to 10 wt.-%, preferably of 0.1 to 5 wt.-% and especially preferably of 1 to 3 wt.-% squalane or squalene or paraffin, each based on the total weight of the topical composition.

Moreover preferably, the at least one buffering agent is selected from the group consisting of lactate, acetate or citrate buffering agents. These buffers allow the solution to be in a pH range of pH 4.5 to pH 6.0, even if benzoyl peroxide is incorporated, which - without buffering agent - would acidify the composition.

In this context, preferably, the topical composition comprises the at least one buffering agent in an amount sufficient to set the pH of the topical composition to a pH value of between 4.5 to 6.0, preferably of between 4.7 to 5.5, especially preferably of between 4.8 to 5.2 and moreover preferably to a pH value of 5.0.

Furthermore preferably, the at least one moisturizing agent of the topical composition is selected from the group consisting of alcohol or alkandiol, preferably of glycerol, propylenglycerol, 1,2-pentanediol, stearyl alcohol, cetyl alcohol, polyoxyethylene cetyl ether, cocoyl caprylocaprate, 1,2,6-hexanetriol and isopropyl myristate.

Also preferably, the at least one moisturizing agent is contained in an amount of between 0.01 to 10 wt.-%, preferably of 0.1 to 7.5 wt.-% and especially preferably of 1 to 6 wt.-%, each based on the total weight of the topical composition.

Furthermore preferably, the topical composition according to the invention additionally comprises at least one adsorbent, preferably selected from the group consisting of silica compounds, preferably bioactivated silicium, mesoporous silica, silicon dioxide, colloid silica and magnesium aluminium silicate, preferably wherein the adsorbent is present in an amount of between 0.001 to 3 wt.-%, preferably of between 0.01 to 3 wt.-%, especially preferably of between 0.1 to 2 wt.-%, based on the total weight of the topical composition.

It was surprisingly found that the incorporation of an adsorbent to the topical formulation was able to further enhance the stability of the topical formulation.

Moreover preferably, nadifloxacin or a pharmaceutically acceptable salt thereof is present in the topical composition in an amount of between 0.5 to 5 wt.-%, preferably of between 0.75 to 2.5 wt.-% and especially preferably of 1 wt.-% dependent on the total weight of the topical composition.

Preferably, benzoyl peroxide is present in an amount of between 0.1 to 10 wt.-%, preferably of between 0.5 to 7.5 wt.-%, moreover preferably of between 1 to 6 wt.-% and especially preferably of 5 wt.-%, based on the total weight of the topical composition.

In another aspect of the invention, the topical composition is used in the treatment of an inflammatory skin disease.

Preferably, the inflammatory skin disease is selected from acne vulgaris, acne comedonica, acne papulo-pustulosa, acne conglobata, acne fulminans, acne neonatorum, acne infantum, acne tarda, acne inversa, acne aestavalis, acne venenata, acne cosmetic, acne medicamentosa or rosaceae. Especially preferable, the inflammatory skin disease is acne vulgaris.

In yet another aspect of the present invention, a method for manufacturing a topical composition is provided, comprising the steps of:
(a) providing at least one gel forming agent, in an amount of between 0.01 to 10 wt.-%, preferably of between 0.1 to 5 wt.-% and especially preferably of between 1 to 4 wt.-%, based on the total weight of the topical composition to be prepared, and optionally a lipid, especially preferably squalane or paraffin,
   and/or at least one emulsifier selected from the group consisting of glyceryl monostearate, PEG-75 stearate, PEG-6 stearate, ethylene glycol stearate, PEG-32 stearate, cetyl alcohol and ethoxylated fatty alcohols, diisostearique,macrogol 30 dipolyhydroxystearate and triglycerol diisostearate,
   and/or at least one surfactant selected from the group consisting of caprylocaproyl polyoxyl-8 Glycerides, polysorbate 20, polysorbate60, polysorbate 80, propylene glycol isostearate
   and/or at least one adsorbent, preferably selected from the group consisting of silica compounds, preferably bioactivated silicium, mesoporous silica, silicon dioxide, colloid silica and magnesium aluminium silicate, preferably wherein the adsorbent is present in an amount of between 0.001 to 3 wt.-%, preferably of between 0.01 to 3 wt.-%, especially preferably of between 0.1 to 2 wt.-%, based on the total weight of the topical composition to be prepared,
   and/or at least one solubiliser selected from the group consisting of diethylene glycol monoethyl ether, poloxamer and propylene glycol monolaurate,
   and/or at least one excipient selected from the group consisting of propylene glycol monolaurate, ethylcellulose and propylene glycol isostearate, in an amount of between 0.01 to 10 wt.-%, preferably of between 0.1 to 5 wt.-% and especially preferably of between 1 to 3 wt.-%, based on the total weight of the topical composition to be prepared,
   and/or at least one moisturizing agent
   and water
(b) providing nadifloxacin and benzoyl peroxide;
(c) combining, preferably mixing, the ingredients as provided in step a) with nadifloxacin and benzoyl peroxide under the use of a buffer system, optionally wherein the gel forming agent is added after nadifloxacin and benzoyl peroxide are evenly distributed in the mixture;
(d) obtaining a topical composition, preferably a topical composition according to the invention.

Preferably, the method comprises the steps of:
a) Providing water and at least one excipient selected from the group consisting of propylene glycol monolaurate, ethylcellulose and propylene glycol isostearate, in an amount of between 0.01 to 10 wt.-%, preferably of between 0.1 to 5 wt.-% and especially preferably of between 1 to 3 wt.-%, based on the total weight of the topical composition to be prepared and mixing,
b) Providing at least one adsorbent, preferably selected from the group consisting of silica compounds, preferably bioactivated silicium, mesoporous silica, silicon dioxide, colloid silica, magnesium aluminium silicate, preferably wherein the adsorbent is present in an amount of between 0.001 to 3 wt.-%, preferably of between 0.01 to 3 wt.-%, especially preferably of between 0.1 to 2 wt.-%, based on the total weight of the topical composition and dispersing the adsorbent in the mixture as obtained by step a),
c) Adding nadifloxacin and benzoyl peroxide into the mixture as obtained by step b) and mixing until homogenously distributed,
d) Adding at least one buffering agent, preferably selected from the group consisting of lactate, acetate or citrate buffering agents, to the mixture as obtained in step c);
e) Adding at least one gel forming agent, preferably selected from the group consisting of xanthan gum, co-polymers of taurate and acrylate, carbomer, modified maize starch, pregelatinised starch, microcrystalline cellulose, guar gum, acrylamido-2-methylpropanesulfonic acid, hydroxyethyl acrylate / sodium acryloyl dimethyl taurate copolymer, polyacrylate crosspolymer-6, crosslinked homopolymer based on 2- acrylamido-2-methylpropane sulfonic acid, copolymer based on 2-acrylamido- 2-methylpropane sulfonic acid and ethoxylated behenyl alcohol methacrylate, crosslinked copolymer based on 2-acrylamido-2-methylpropane sulfonic acid and N-vinylpyrrolidone, copolymer based on acrylamide/sodium acryloyldimethyl taurate /isohexadecane/ polysorbate 80 to the mixture as obtained in step d),
f) Stirring the mixture as obtained in step e) until the gel is formed,
g) Obtaining a topical composition according to the invention.

Preferably, the gel forming agent is added in an amount of from of between 0.01 to 10 wt.-%, preferably of between 0.1 to 5 wt.-% and especially preferably of between 1 to 4 wt.-%, based on the total weight of the topical composition to be prepared.

Preferably, the method is conducted at a temperature of between 4 to 30°C, preferably of between 8 to 25 °C and especially preferably of between 10 to 25 °C.

Preferably, the method according to the invention comprises the following steps:
1) preparing a carrier mixture, comprising the steps of:
   (a-1) providing at least one gel forming agent, in an amount of between 0.01 to 10 wt.-%, preferably of between 0.1 to 5 wt.-% and especially preferably of between 1 to 4 wt.-%, based on the total weight of the topical composition to be prepared,
      and preferably a lipid, especially preferably squalane or paraffin,
      and/or at least one emulsifier selected from the group consisting of glyceryl monostearate, PEG-75 stearate, PEG-6 stearate, ethylene glycol stearate, PEG-32 stearate, cetyl alcohol and ethoxylated fatty alcohols, diisostearate, macrogol30 dipolyhydroxystearate and triglycerol diisostearate, and/or at least one surfactant selected from the group consisting of caprylocaproyl polyoxyl-8 glycerides, polysorbate 20, polysorbate 60, polysorbate 80, propylene glycol Isostearate,
      and/or at least one solubiliser selected from the group consisting of Diethylene glycol monoethyl ether, poloxamer and propylene glycol monolaurate,
      and/or at least one excipient selected from the group consisting of 15 Propylene glycol monolaurate, ethylcellulose and propylene glycol isostearate,
      in an amount of between 0.01 to 10 wt.-%, preferably of between 0.1 to 5 wt.-% and especially preferably of between 1 to 3 wt.-%, based on the 20 total weight of the topical composition to be prepared;
   (b-1) providing at least one moisturizing agent and water;
   (c-1) combining the two components provided in step (a) and (b) by mixing and additionally adding at least one buffering agent, preferably lactate, acetate or citrate;
   (d-1) obtaining a carrier mixture;
2) introducing nadifloxacin and benzoyl peroxide into the carrier mixture prepared in step 1), comprising the steps of:
   (a-2) providing nadifloxacin and benzoyl peroxide;
   (b-2) providing an amount of 5 to 50 wt.-%, preferably 10 to 50 wt.-% and especially preferably of 25 wt.-%, based on the total amount of the topical composition to be prepared, of the carrier mixture as prepared in step 1).
   (c-2) introducing nadifloxacin and benzoyl peroxide provided in step (a-2) into the carrier mixture provided in step (b-2) and adding the remaining amount after step (b-2) of the carrier mixture to the mixture;
   (d-2) combining the carrier mixture with nadifloxacin and benzoyl peroxide by incorporating the mixtures into each other;
   (e-2) homogenizing the combined mixture as obtained in step (d-2), preferably wherein the homogenization is achieved by using a rotor-stator system, a high pressure homogenizer, a pump or a static mixer;
   (f-2) obtaining a topical composition, preferably a topical composition according to the invention.

The invention is further described by the following illustrative, non-limiting examples.

### Short description of figures

**Figure 1****:** Depiction of the amount of active ingredient at different time points and temperatures using the gel forms Amaze^{®} XT (Dehydro xanthan gum), Ketrol^{®} F (Xanthan gum), SEPINEO^{®} D.E.R.M (Hydroxyethyl Acrylate/Sodium Acryloyldimethyl Taurate Copolymer) and SEPINOV^{®} EMT (Hydroxyethyl Acrylate/Sodium Acryloyldimethyl Taurate Copolymer).
**Figure 2****:** Depiction of the influence of different buffer systems on the amount of active ingredient at different time points and temperatures.
**Figure 3****:** Depiction of the active ingredients (API) content in prototype F (see example 2) formulation at different time points and temperatures. NDN is nadifloxacin and BPO is benzoyl peroxide.
**Figure 4****:** Depiction of the active ingredients (API) content in prototype G (see example 2) formulation at different time points and temperatures. NDN is nadifloxacin and BPO is benzoyl peroxide.
**Figure 5****:** Depiction of the active ingredients (API) content in prototype H (see example 2) formulation at different time points and temperatures. NDN is nadifloxacin and BPO is benzoyl peroxide.

### Examples

### Example 1 - Stability of benzoyl peroxide in combination with different gel forming agents, additives and buffer systems

### A) Evaluation of different gel forming agents on the stability of benzoyl peroxide

Compositions of 10 % benzoyl peroxide and the gel forming agents (value in bracket is the amount used or the test compositions) Amaze^{®}XT (xanthan gum, 2 wt.-%), Ketrol^{®} F (xanthan gum, 3 wt.-%), Sepineo^{®} D.E.R.M. (Copolymer of Hydroxyethyl Acrylate/Sodium Acryloyldimethyl Taurate, 2 wt.-%) and SEPINOV^{®} EMT (Copolymer of ydroxyethyl Acrylate/Sodium Acryloyldimethyl Taurate, 2 wt.-%) were prepared and evaluated for their influence on the stability of BPO.

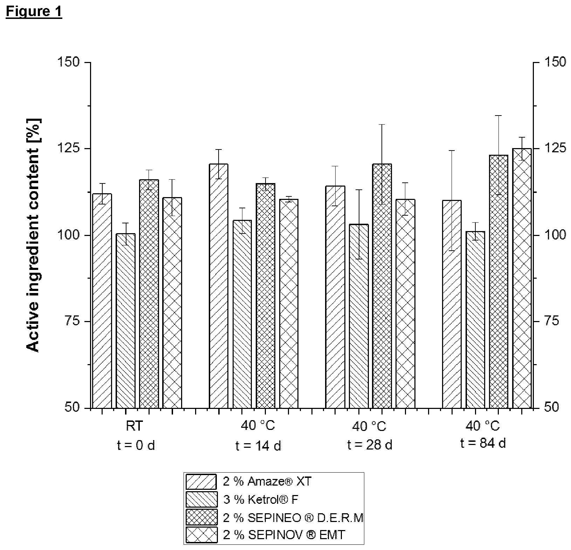

The results are shown in Figure 1. It could be observed that benzoyl peroxide is stable (> 95 wt.-% of initial benzoyl peroxide amount) in all used gel forming agents for at least 84 days at 40 °C.

### B) Evaluation of the influence of different buffer systems on the stability of benzoyl peroxide

Compositions of 10 % benzoyl peroxide and the buffers phosphate buffer (Disodium monohydrogen phosphate-Anhydrate/ sodium dihydrogen phosphate dihydrate), Acetic Acid buffer, sodium hydroxide and lactic acid - lactate - buffer were prepared and evaluated for the observed pH decrease.

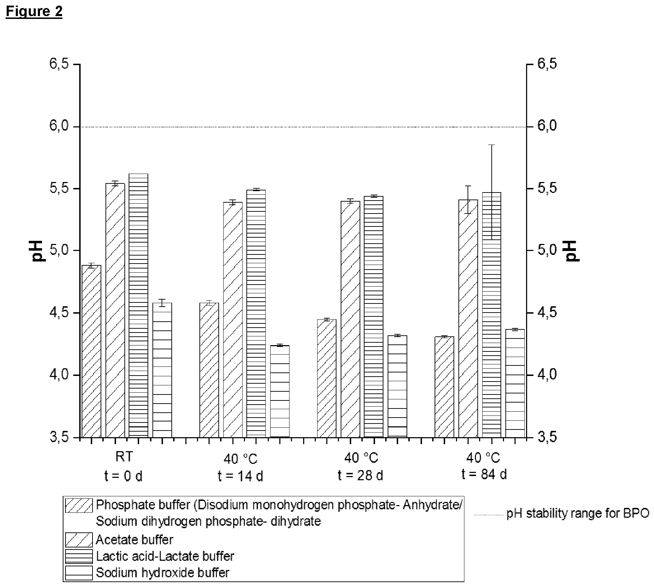

The results are shown in Figure 2. It could be observed that the buffer systems acetic acid - acetate buffer and lactic acid - lactate buffer is able to retain the pH value at ~ pH 5.5 over a time of 84 days and a temperature of 40°C.

### Example 2 - Evaluation of the stability of a topical composition with nadifloxacin and benzoyl peroxide over 84 days

Three different compositions with 1 wt.-% nadifloxacin and 5 wt.-% benzoyl peroxide were prepared (Table 1) and evaluated for their API stability.

**Table 1 - Overview of different compositions. Each composition comprises 1 wt.-% nadifloxacin and 5 wt.-% benzoyl peroxide.**

| **Composition F** | | **Composition G** | | **Composition H** | |
|---|---|---|---|---|---|
| Component | Amount [wt.-%] | Component | Amount [wt.-%] | Component | Amount [wt.-%] |
| Xanthan gum | 1.0 | Xanthan gum | 1.0 | Microcrystalline cellulose and xanthan gum | 4.0 |
| Polysorbate 60 | 0.5 | Silica | 0.5 | - | - |
| Squalane | 2.5 | - | - | Squalane | 2.5 |
| 1,2-pentanediol | 5.0 | 1,2-pentanediol | 5.0 | 1,2-Pentanediol | 5.0 |
| Lactate buffer | q.s. pH 5.5 | Lactate buffer | q.s. pH 5.5 | Lactate buffer | q.s. pH 5.5 |
| Purified water | ad 100 | Purified water | ad 100 | Purified water | ad 100 |

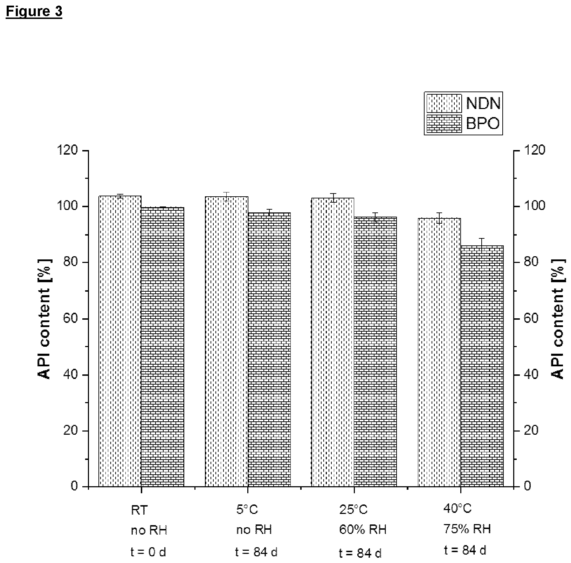

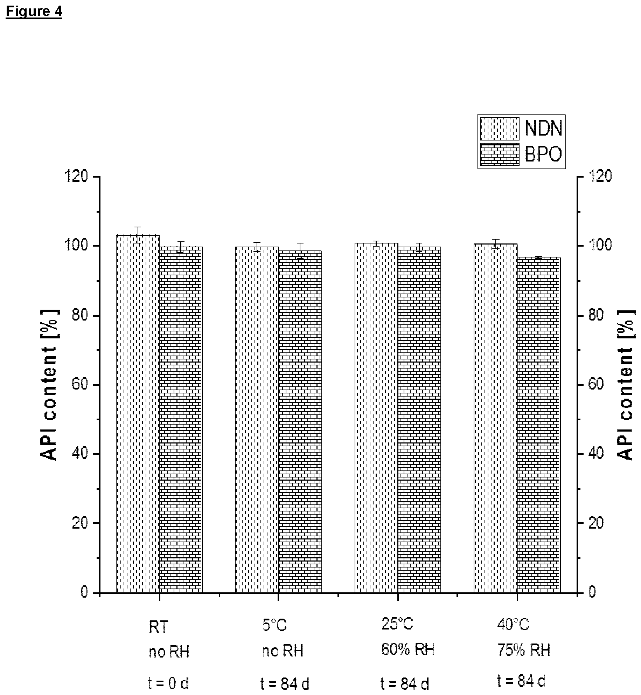

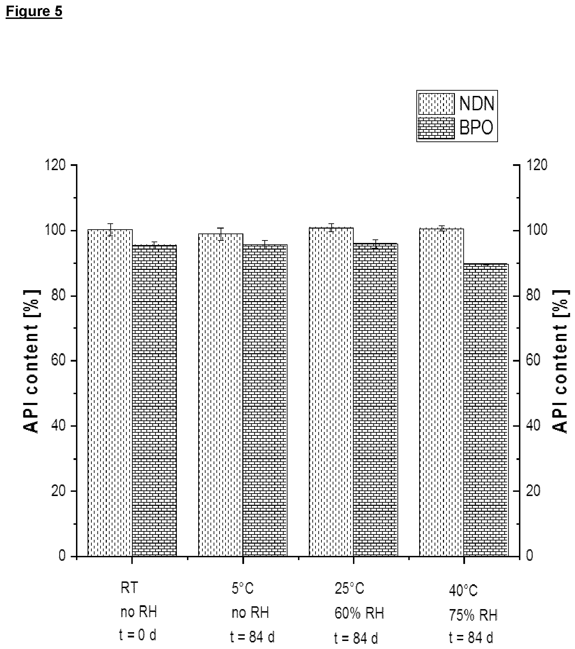

The results are shown in Figure 3, 4 and 5. It can be observed that all compositions show a high stability (> or ~ 95 wt.-% API content in comparison to the initial content) after 84 days, wherein the composition G shows a high stability even at a humidity of 75 % and a storage temperature of 40°C.

### Example 3 - Preparation of topical composition

A topical composition is prepared using the following steps:
1. 70 mL water and 6 mL pentylene glycol are mixed using an anchor stirrer.
2. 1 g mesoporous silicia is added and dispersed in the water / pentylene glycol mixture of step 1 using an Ultra Turrax and an anchor stirrer.
3. Adding 1 wt.-% nadifloxacin and 5 wt.-% benzoyl peroxide as APIs dependent on the total amount of topical composition to be prepared.
4. Mixing with an anchor stirrer and an Ultra Turrax until both APIs are homogeneously distributed.
5. Adding lactate buffer (0.05 M) until a pH of 5.4 - 5.5 is reached.
6. Adding 3 g of a crosslinked homopolymer based on 2-acrylamido-2-methylpropane sulfonic acid (Motusflex MF, Clariant AG).
7. Stirring the mixture until a gel is formed.

### Example 4 - Interaction of formulation with sebum model

The hair follicle canal is often blocked with sebum in patients with acne. It is thus evaluated how a topical composition as produced in examples 2 or 3 interacts with sebum to deliver the active substances to the microorganisms causing the inflammation under the sebum plug.

An experimental set-up was built to test the sebum penetration. A PTFE filter membrane is loaded with artificial sebum comprising a UV active colorant and the topical formulation according to examples 2 or 3 is loaded thereto and removed after 24 hours of incubation time. Depending on the strength of interaction between artificial sebum and topical composition, an amount of the artificial sebum is removed, which can be quantified with the UV active colorant. The remaining artificial sebum is removed and analyzed via HPLC to determine the grade of interaction between sebum and topical composition.

The topical composition according to the invention was able to penetrate the artificial sebum, wherein the penetration was predominantly by benzoyl peroxide. Thus, it was proven that a combination of both ingredients is beneficial for serum penetration and thus for delivering nadifloxacin to the inflammation causing microorganisms.

## Claims

1. Topical composition comprising:
i) nadifloxacin or a pharmaceutically acceptable salt thereof,
ii) benzoyl peroxide,
iii) at least one gel forming agent, preferably selected from the group consisting of xanthan gum, co-polymers of taurate and acrylate, carbomer, modified maize starch, pregelatinised starch, microcrystalline cellulose, guar gum, acrylamido-2-methylpropanesulfonic acid, hydroxyethyl acrylate / sodium acryloyl dimethyl taurate copolymer, polyacrylate crosspolymer-6, crosslinked homopolymer based on 2- acrylamido-2-methylpropane sulfonic acid, copolymer based on 2-acrylamido- 2-methylpropane sulfonic acid and ethoxylated behenyl alcohol methacrylate, crosslinked copolymer based on 2-acrylamido-2-methylpropane sulfonic acid and N-vinylpyrrolidone and copolymer based on acrylamide/sodium acryloyldimethyl taurate /isohexadecane/ polysorbate 80, preferably in an amount of from 0.01 to 10 wt.-%, preferably of from 0.1 to 5 wt.-% and especially preferably in an amount of from 1 to 4 wt.-%, based on the total weight of the topical composition,
iv) at least one buffering agent,
v) at least one moisturizing agent.

2. Topical composition according to claim 1 further comprising a lipid phase, preferably, wherein the lipid phase comprises squalane or paraffin
and/or at least one emulsifier selected from the group consisting of glyceryl monostearate, PEG-75 stearate, PEG-6 stearate, ethylene glycol stearate, PEG-32 stearate, cetyl alcohol and ethoxylated fatty alcohols, diisostearate, macrogol30 dipolyhydroxystearate and triglycerol diisostearate,
and/or at least one surfactant selected from the group consisting of caprylocaproyl polyoxyl-8 glycerides, polysorbate 20, polysorbate 60, polysorbate 80, propylene glycol isostearate solubiliser: diethylene glycol monoethyl ether, poloxamer and propylene glycol monolaurate,
and/or at least one excipient selected from the group consisting of propylene glycol monolaurate, ethylcellulose and propylene glycol Isostearate.

3. Topical composition according to claim 2, wherein the lipid phase comprises or consists of 0.01 to 10 wt.-%, preferably of 0.1 to 5 wt.-% and especially preferably of 1 to 3 wt.-% squalane or squalene or paraffin, each based on the total weight of the topical composition.

4. Topical composition according to any one of the preceding claims, wherein the at least one buffering agent is selected from the group consisting of lactate, acetate or citrate buffering agents,
and/or
wherein the topical composition comprises the at least one buffering agent in an amount sufficient to set the pH of the topical composition to a pH value of between 4.5 to 6.0, preferably of between 4.7 to 5.5, especially preferably of between 4.8 to 5.2 and moreover preferably to a pH value of 5.0.

5. Topical composition according to any one of the preceding claims, wherein the at least one moisturizing agent is selected from the group consisting of alcohol or alkandiol, preferably of glycerol, propylenglycerol, 1,2-pentanediol, stearyl alcohol, cetyl alcohol, polyoxyethylene cetyl ether, cocoyl caprylocaprate, 1,2,6-hexanetriol, isopropyl myristate.

6. Topical composition according to any one of the preceding claim, wherein the at least one moisturizing agent is contained in an amount of between 0.01 to 10 wt.-%, preferably of 0.1 to 7.5 wt.-% and especially preferably of 1 to 6 wt.-%, each based on the total weight of the topical composition.

7. Topical composition according to any one of the preceding claims, wherein the topical composition comprises additionally at least one adsorbent, preferably selected from the group consisting of silica compounds, preferably bioactivated silicium, mesoporous silica, silicon dioxide, colloid silica and magnesium aluminium silicate, preferably wherein the adsorbent is present in an amount of between 0.001 to 3 wt.-%, preferably of between 0.01 to 3 wt.-%, especially preferably of between 0.1 to 2 wt.-%, based on the total weight of the topical composition.

8. Topical composition according to any one of the preceding claims, wherein nadifloxacin or a pharmaceutically acceptable salt thereof is present in an amount of between 0.5 to 5 wt.-%, preferably of between 0.75 to 2.5 wt.-% and especially preferably of 1 wt.-% dependent on the total weight of the topical composition.

9. Topical composition according to any one of the preceding claims, benzoyl peroxide is present in an amount of between 0.1 to 10 wt.-%, preferably of between 0.5 to 7.5 wt.-%, moreover preferably of between 1 to 6 wt.-% and especially preferably of 5 wt.-%, based on the total weight of the topical composition.

10. Topical composition according to any one of the previous claims for use in the treatment of an inflammatory skin disease.

11. Topical composition according to any one of the previous claims for use according to claim 10, wherein the inflammatory skin disease is selected from acne vulgaris, acne comedonica, acne papulo-pustulosa, acne conglobata, acne fulminans, acne neonatorum, acne infantum, acne tarda, acne inversa, acne aestavalis, acne venenata, acne cosmetic, acne medicamentosa or rosaceae.

12. Method for manufacturing a topical composition, preferably a topical composition according to any one of the claims 1 to 9, comprising or consisting of the steps:
a) providing at least one gel forming agent, in an amount of between 0.01 to 10 wt.-%, preferably of between 0.1 to 5 wt.-% and especially preferably of between 1 to 4 wt.-%, based on the total weight of the topical composition to be prepared,
and optionally a lipid phase, preferably, wherein the lipid phase comprises squalane or paraffin,
and/or at least one emulsifier selected from the group consisting of glyceryl monostearate, PEG-75 stearate, PEG-6 stearate, ethylene glycol stearate, PEG-32 stearate, cetyl alcohol and ethoxylated fatty alcohols, diisostearique, macrogol 30 dipolyhydroxystearate and triglycerol diisostearate,
and/or at least one surfactant selected from the group consisting of caprylocaproyl polyoxyl-8 Glycerides, polysorbate 20, polysorbate 60, polysorbate 80, propylene glycol isostearate
and/or at least one solubiliser selected from the group consisting of Diethylene glycol monoethyl ether, poloxamer and propylene glycol monolaurate,
and/or at least one excipient selected from the group consisting of Propylene glycol monolaurate, ethylcellulose and propylene glycol isostearate, in an amount of between 0.01 to 10 wt.-%, preferably of between 0.1 to 5 wt.-% and especially preferably of between 1 to 3 wt.-%, based on the total weight of the topical composition to be prepared,
and/or at least one moisturizing agent
and water
b) providing nadifloxacin and benzoyl peroxide;
c) combining, preferably mixing, the ingredients as provided in step a) with nadifloxacin and benzoyl peroxide under the use of a buffer system, optionally wherein the gel forming agent is added after nadifloxacin and benzoyl peroxide are evenly distributed in the mixture;
d) obtaining a topical composition, preferably a topical composition according to any one of claims 1 to 9.

13. Method according to claim 12, wherein step c) is conducted at a temperature of between 4 to 30 °C, preferably of between 8 to 25 °C and especially preferably of between 10 to 25 °C.

14. Topical composition according to any of claims 1 to 9, wherein the composition is obtainable or obtained by a method according to any of claims 12 and 13.

15. Topical composition according to any one of claim 1 to 9 for use according to claim 10 or 11, wherein the composition is obtainable or obtained by a method according to any of claims 12 and 13.
